# EUROPÄISCHE PATENTSCHRIFT

(11) **EP 0 489 299 B1**
(45) Veröffentlichungstag und Bekanntmachung des Hinweises auf die Patenterteilung: **07.09.1994**
(21) Anmeldenummer: 91119701.0
(22) Anmeldetag: 19.11.1991
(51) Int. Cl.: C07C 317/14, C07C 317/32, C07C 317/36, C07C 317/40, C09B 62/447

(54) **Arylsulfonylverbindungen, die ungesättigte Reste aufweisen**
Arylsulfonyl compounds, bearing unsaturated residues
Composés arylsulfonyle, avec des restes insaturés

(30) Priorität: 30.11.1990 DE 4038200
(43) Veröffentlichungstag der Anmeldung: 10.06.1992
(73) Patentinhaber: BASF Aktiengesellschaft, 67063 Ludwigshafen (DE)
(72) Erfinder: Siegel, Bernd, Dr., W-6700 Ludwigshafen (DE); Marschner, Claus, Dr., W-6720 Speyer (DE); Patsch, Manfred, Dr., W-6706 Wachenheim (DE)

(56) Entgegenhaltungen:
- EP-A- 0 392 351
- US-A- 3 420 812
- JOURNAL OF THE CHEMICAL SOCIETY, PERKIN TRANSACTIONS II, Nr. 8, August 1988, Seiten 1377-1383, London, GB; B.D. GUPTA et al.: "Homolytic displacement at carbon. Part. 3. First example of alpha-attack on the allenyl- and prop-2-ynyl-cobaloximes"
- BULLETIN OF THE CHEMICAL SOCIETY OF JAPAN, Band 62, Nr. 10, Oktober 1989, Seiten 3349-3354, Tokyo, JP; D.V. RAMANA et al.: "Ortho effects in organic molecules on electron impact. 20. Parallel oxygen transfers from nitro group to sulfur and acetylenic triple bond in 3-(o-nitrophenylthio)-1-propynes"
- CHEMICAL ABSTRACTS, Band 56, Nr. 6, 19. März 1962, Spalte 5872, Zusammenfassung Nr. 5872g-5873e, Columbus, Ohio, US; L. MAIOLI et al.: "Nucleophilic substitution in ethylenic derivatives. VI. 1-Arylsulfonyl-2-methyl-2-haloethylenes"
- CHEMICAL ABSTRACTS, Band 112, Nr. 13, 26. März 1990, Seite 693, Zusammenfassung Nr. 118372g, Columbus, Ohio, US; V.N. MIKHAILOVA et al.: "Hydration of 1- and 3-(arylsulfonyl)-1-propynes and (arylsulfonyl)allenes"
- CHEMICAL ABSTRACTS, Band 71, Nr. 25, 22. Dezember 1969, Seite 392, Zusammenfassung Nr. 123806r, Columbus, Ohio, US; V.N. MIKHAILOVA et al.: "Aryl dihalopropyl sulfides and sulfones, their synthesis and properties",
- CHEMICAL ABSTRACTS, Band 107, Nr. 21, 23. November 1987, Seite 701, Zusammenfassung Nr. 197678j, Columbus, Ohio, US; V.V. YAKOLEV et al.: "Disulfone formation in the reaction of sodium arenesulfinates with 4-chloro-2-butyn-1-o1"

## Beschreibung

Die vorliegende Erfindung betrifft neue Sulfonylverbindungen der Formel I 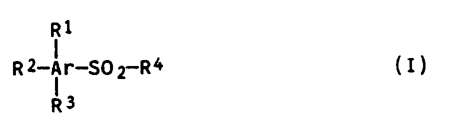
in freier Form oder in Form ihrer Salze, worin
- Ar: den Rest eines Benzol- oder Naphthalinringes,
- R¹ und R²: gleich oder verschieden sind und unabhängig voneinander jeweils Wasserstoff, gegebenenfalls durch Amino oder C₁-C₄-Alkanoylamino substituiertes C₁-C₄-Alkyl, gegebenenfalls substituiertes Phenyl, C₁-C₄-Alkoxy, Carboxyl, C₁-C₄-Alkoxycarbonyl, Cyano, Halogen oder Hydroxysulfonyl,
- R³: Amino, C₁-C₄-Alkanoylamino oder Benzoylamino und
- R⁴: einen Rest der Formel 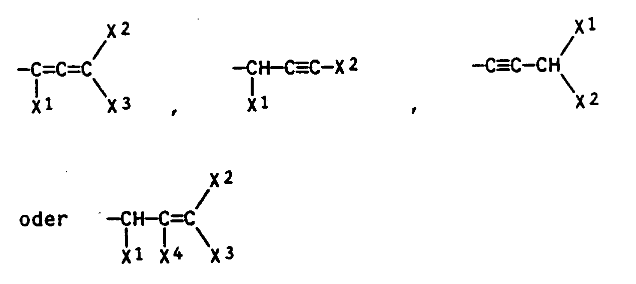 bedeuten, worin
- X¹, X² und X³: gleich oder verschieden sind und unabhängig voneinander jeweils für Wasserstoff, gegebenenfalls durch Hydroxy oder C₁-C₄-Alkoxy substituiertes C₁-C₄-Alkyl oder gegebenenfalls substituiertes Phenyl und
- X⁴: für eine unter alkalischen Reaktionsbedingungen abspaltbare Gruppe stehen,
deren Herstellung sowie die Verwendung derjenigen Sulfonylverbindungen, in denen R³ Amino bedeutet, für die Synthese von Reaktivfarbstoffen.

Aufgabe der vorliegenden Erfindung war es, neue Arylsulfonylverbindungen bereitzustellen, die über ungesättigte Reste verfügen und die sich vorteilhaft als Anker für Reaktivfarbstoffe und, für den Fall, daß sie zusätzlich eine Aminogruppe aufweisen, auch als Diazokomponente eignen sollten. Außerdem sollte ein geeignetes Herstellverfahren bereitgestellt werden, das die neuen Verbindungen in vorteilhafter Weise liefert.

Demgemäß wurden die eingangs näher bezeichneten Sulfonylverbindungen der Formel I gefunden.

Alle in der obengenannten Formel I auftretenden Alkylgruppen können sowohl geradkettig als auch verzweigt sein.

Wenn in der obengenannten Formel I substituierte Phenylreste auftreten, so kommen als Substituenten C₁-C₄-Alkyl, C₁-C₄-Alkoxy oder Halogen in Betracht. Die Phenylreste sind dabei üblicherweise einbis dreifach substituiert.

Reste R¹, R², X¹, X² und X³ sind z.B. Methyl, Ethyl, Propyl, Isopropyl, Butyl, Isobutyl, sec-Butyl, Phenyl, 2- oder 4-Methylphenyl, 2,4-Dimethylphenyl, 2- oder 4-Chlorphenyl, 2,4-Dichlorphenyl, 2- oder 4-Methoxyphenyl oder 2,4-Dimethoxyphenyl.

Reste R¹ und R² sind weiterhin z.B. Fluor, Chlor, Brom, Methoxy, Ethoxy, Propoxy, Isopropoxy, Butoxy, Isobutoxy, sec-Butoxy, Aminomethyl, 2-Aminoethyl, 2- oder 3-Aminopropyl, 2- oder 4-Aminobutyl, Formylaminomethyl, Acetylaminomethyl, Propionylaminomethyl, 2-Formylaminoethyl, 2-Acetylaminoethyl, 2-Propionylaminoethyl, 2- oder 3-Formylaminopropyl, 2- oder 3-Acetylaminopropyl, 2- oder 3-Propionylaminopropyl, 2- oder 4-Formylaminobutyl, 2- oder 4-Acetylaminobutyl, 2- oder 4-Propionylaminobutyl, Methoxycarbonyl, Ethoxycarbonyl, Propoxycarbonyl, Isopropoxycarbonyl oder Butoxycarbonyl.

Reste R³ sind weiterhin z.B. Formylamino, Acetylamino, Propionylamino, Butyrylamino oder Isobutyrylamino.

Reste X¹, X² und X³ sind weiterhin z.B. Hydroxymethyl, 2-Hydroxyethyl, 2- oder 3-Hydroxypropyl, 2- oder 4-Hydroxybutyl, Methoxymethyl, Ethoxymethyl, 2-Methoxyethyl, 2-Ethoxyethyl, 2- oder 3 Methoxypropyl, 2- oder 3-Ethoxypropyl, 2- oder 4-Methoxybutyl oder 2- oder 4-Ethoxybutyl.

X⁴ in Formel I steht für eine unter alkalischen Reaktionsbedingungen abspaltbare Gruppe. Solche Gruppen sind z.B. Halogen, wie Chlor, Brom oder Iod, OSO₃H, SSO₃H, OP(O)(OH)₂, C₁-C₄-Alkylsulfonyloxy, gegebenenfalls substituiertes Phenylsulfonyloxy, C₁-C₄-Alkanoyloxy, C₁-C₄-Dialkylamino oder ein Rest der Formel 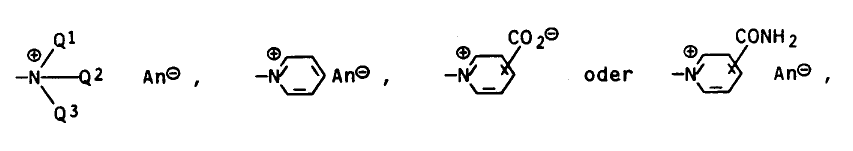
wobei Q¹, Q² und Q³ gleich oder verschieden sind und unabhängig voneinander jeweils die Bedeutung von C₁-C₄-Alkyl oder Benzyl und An^{⊖} jeweils die Bedeutung eines Anions besitzen. Als Anion An^{⊖} kann dabei z.B. Fluorid, Chlorid, Bromid, Iodid, Mono-, Di- oder Trichloracetat, Methylsulfonat, Phenylsulfonat oder 2- oder 4-Methylphenylsulfonat in Betracht kommen. Vorzugsweise bedeutet X⁴ Halogen.

Die Sulfonylverbindungen der Formel I können entweder in freier Form oder in Form ihrer Salze vorliegen. Wenn sie in Salzform vorliegen, können sie, sofern sie über Hydroxysulfonyl- oder Carboxylgruppen verfügen, beispielsweise in Form der Alkalisalze, z.B. der Lithium-, Natrium- oder Kaliumsalze, oder der Ammoniumsalze vorliegen.

Wenn sie über Aminogruppen verfügen, können sie beispielsweise in Form der Halogenide, wie Fluorid, Chlorid, Bromid oder Iodid, oder als Hydrogensulfat oder Sulfat vorliegen.

Wenn sie sowohl über Hydroxysulfonyl- oder Carboxylgruppen als auch über Aminogruppen verfügen, können sie auch in Betain-Form vorliegen.

Hervorzuheben sind Sulfonylverbindungen der Formel I, in der einer der beiden Reste R¹ und R² Wasserstoff und der andere ebenfalls Wasserstoff oder gegenenfalls durch Amino oder C₁-C₄-Alkanoylamino substituiertes C₁-C₄-Alkyl, C₁-C₄-Alkoxy oder Halogen bedeutet.

Bevorzugt sind Sulfonylverbindungen der Formel I, in der R⁴ einen Rest der Formel 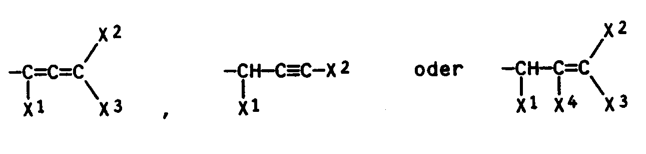
bedeutet, worin
- X¹, X² und X³: jeweils für Wasserstoff und
- X⁴: für Halogen stehen.

Besonders bevorzugt sind Sulfonylverbindungen der Formel I, in der R⁴ einen Rest der Formel 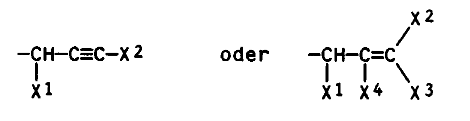
bedeutet, worin
- X¹ X² und X³: jeweils für Wasserstoff und X⁴ für Halogen stehen.

Es wurde weiterhin gefunden, daß man Sulfonylverbindungen, die der Formel Ia 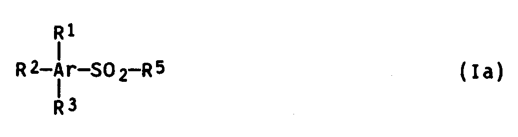
gehorchen, worin Ar, R¹, R² und R³ jeweils die obengenannte Bedeutung besitzen und R⁵ einen Rest der Formel 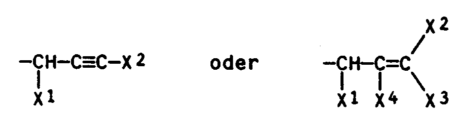
bedeutet, worin X¹, X², X³ und X⁴ jeweils die obengenannte Bedeutung besitzen, durch Umsetzung von Arylsulfinsäuren der Formel II 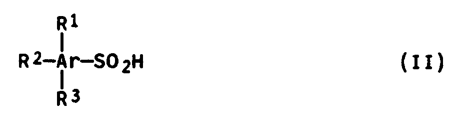
mit Halogenverbindungen der Formel IIIa oder IIIb 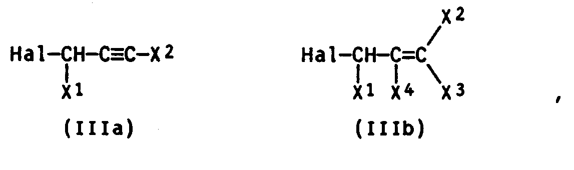
wobei Hal jeweils Halogen bedeutet und Ar, R¹, R², R³, X¹, X², X³ und X⁴ jeweils die obengenannte Bedeutung besitzen, in wäßrigem Medium bei einer Temperatur von 20 bis 95°C vorteilhaft erhält, wenn man die Umsetzung bei einem pH-Wert von 2,5 bis 7, vorzugsweise 4 bis 6,5, durchführt.

Das erfindungsgemäße Verfahren wird in der Regel in Wasser oder in einem Gemisch aus Wasser und einem mit Wasser mischbaren Lösungsmittel vorgenommen. Geeignete wassermischbare Lösungsmittel sind z.B. C₁-C₄-Alkanole, wie Methanol, Ethanol, Propanol, Isopropanol, Butanol oder Isobutanol, N,N-Dimethylformamid, N-Methylpyrrolidinon, Tetrahydrofuran oder Aceton.

Zweckmäßig legt man dabei die Arylsulfinsäure II in Wasser oder in einem Gemisch aus Wasser und einem wassermischbaren Lösungsmittel vor und gibt dann die Halogenverbindung IIIa oder IIIb zu.

Der pH-Wert wird im allgemeinen mittels Zugabe von Base, z.B. Natrium- oder Kaliumcarbonat, eingestellt, da die Arylsulfinsäuren stark sauer reagieren.

Der Vorteil des neuen Verfahrens ist darin zu sehen, daß im Vergleich zu bekannten Verfahren, die im basischen Milieu durchgeführt werden, beim Arbeiten in saurem Medium die Bildung von Nebenprodukten zurückgedrängt wird.

Aus den im erfindungsgemäßen Verfahren resultierenden Sulfonylverbindungen der Formel Ia können dann diejenigen Sulfonylverbindungen, in denen R⁴ einen Rest der Formel 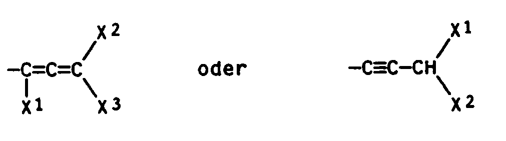
bedeutet, worin X¹, X² und X³ jeweils die obengenannte Bedeutung besitzen, z.B. durch Behandlung mittels Base erhalten werden (siehe z.B. Houben-Weyl "Methoden der Organischen Chemie", Band V/2a, Seiten 975 und 1074).

Die neuen Sulfonylverbindungen der Formel I sind wertvolle Zwischenprodukte für die Synthese von Farbstoffen.

Insbesondere können diejenigen Sulfonylverbindungen der Formel Ib 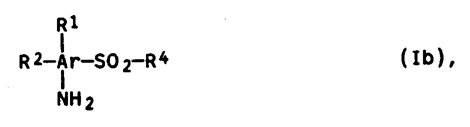
in der R¹, R² und R⁴ jeweils die obengenannte Bedeutung besitzen, als Ankersystem und gegebenenfalls gleichzeitig als Diazokomponente bei der Herstellung von Reaktivfarbstoffen Verwendung finden.

Die folgenden Beispiele sollen die Erfindung näher erläutern.

### Beispiel 1

Eine Lösung von 398 g p-Acetylaminobenzolsulfinsäure in 1000 ml Wasser wurde mit Natriumcarbonat auf einen pH-Wert von 7 gestellt. Diese Lösung wurde innerhalb von 8 Stunden in eine 60°C warme Mischung aus 444 g 2,3-Dichlorprop-1-en, 500 ml Tetrahydrofuran (THF) und 500 ml Wasser getropft. Das Reaktionsgemisch wurde weitere 4 Stunden bei 60°C nachgerührt. Während der gesamten Reaktionszeit wurde der pH-Wert durch Zutropfen von 5 gew.-%iger Natriumbicarbonatlösung bei 6,5 bis 6,9 gehalten. Nach dem Abkühlen wurde von unlöslichen Bestandteilen abfiltriert, das Filtrat mit Kochsalz gesättigt und mehrmals mit Essigester extrahiert. Nach dem Abziehen des Lösungsmittels unter vermindertem Druck isolierte man 163,5 g der Verbindung der Formel 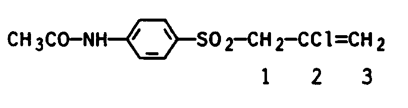
- ¹H-NMR (D₆-DMS0): δ =: 2,1 (s, 3H, CH₃); 4,5 (s, 2H, 1-H); 5,4 (s, 1H, 3-H); 5,6 (s, 1H, 3-H); 7,8 (brs, 4H, Aromaten-H); 10,4 (s, 1H, NH) ppm.

### Beispiel 2

40 g der Verbindung aus Beispiel 1 wurden in 500 g halbkonz. Salzsäure 7 Stunden auf 60°C erhitzt. Die Reaktionslösung wurde anschließend unter vermindertem Druck bei 50°C eingeengt. Man isolierte 39 g der Verbindung der Formel 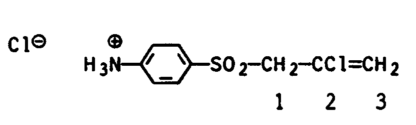
- ¹H-NMR (D₆-DMS0): δ =: 4,3 (s, 2H, 1-H); 5,4 (s, 1H, 3-H); 5,5 (s, 1H, 3-H); 6,9 (d, 2H, Aromaten-H); 7,6 (d, 2H, Aromaten-H); 8,3 (brs, 3H, NH₃^{⊕}) ppm.

### Beispiel 3

Eine Lösung von 199 g p-Acetylaminobenzolsulfinsäure in 500 ml Wasser wurde mit Natriumcarbonat auf einen pH-wert von 7 gestellt. Diese Lösung wurde innerhalb von 1 Stunde in eine 40 bis 45°C warme Mischung aus 219,8 g 2,3-Dibromprop-1-en, 250 ml THF und 250 ml Wasser getropft. Das Reaktionsgemisch wurde weitere 3 Stunden bei 40 bis 45°C nachgerührt.

Während der gesamten Reaktionszeit wurde der pH-Wert durch Zutropfen von 5 gew.-%iger Natriumbicarbonatlösung bei 6,5 bis 6,9 gehalten. Nach dem Abkühlen wurde analog Beispiel 1 aufgearbeitet. Man isolierte 207 g der Verbindung der Formel 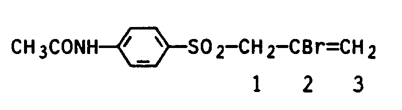
- ¹H-NMR (D₆-DMS0): δ =: 2,1 (s, 3H, CH₃); 4,5 (s, 2H, 1-H); 5,7 (s, 1H, 3-H); 5,9 (s, 1H, 3-H); 7,9 (brs, 4H, Aromaten-H); 10,3 (brs, 1H, NH) ppm.

### Beispiel 4

32 g der Verbindung aus Beispiel 3 wurden in 300 g halbkonz. Salzsäure analog Beispiel 2 hydrolysiert. Man isolierte 31 g einer Substanz, die nach NMR-Spektroskopie folgende Struktur besitzt. 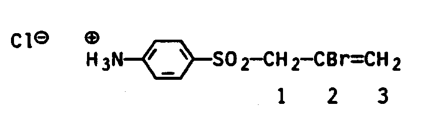
- ¹H-NMR (D₆-DMS0): δ =: 4,4 (s, 2H, 1-H); 5,7 (s, 1H, 3-H); 5,8 (s, 1H, 3-H); 6,85 (d, 2H, Aromaten-H); 7,6 (d, 2H, Aromaten-H), 8,3 (brs, 3H, NH₃^{⊕}) ppm.

Analog den Beispielen 1 und 2 werden die in der folgenden Tabelle 1 aufgeführten Verbindungen der Formel 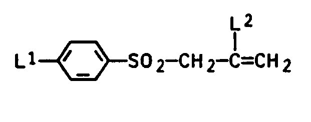
erhalten.

**Tabelle 1**

| Bsp. Nr. | L¹ | L² |
|---|---|---|
| 5 | CH₃CO-NHCH₂CH₂ | Cl |
| 6 | Cl^{⊖}H₃N^{⊕}CH₂CH₂ | Cl |

### Beispiel 9 (nicht beansprucht)

187 g m-Nitrobenzolsulfinsäure wurden bei einem pH-Wert von 7 in 1 l Wasser gelöst und innerhalb von 4 Stunden zu einer 80°C warmen Mischung aus 222 g 2,3-Dichlorprop-1-en in 200 ml Wasser getropft. Man rührte 8 Stunden bei 80°C nach und ließ dabei den pH-Wert auf ca. 2,5 fallen. Nach dem Abkühlen wurde der Niederschlag abgesaugt und unter vermindertem Druck bei 50°C getrocknet. Man isolierte 209 g der Verbindung der Formel 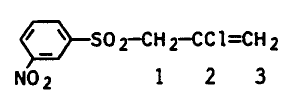
- ¹H-NMR (D₆-DMS0): δ =: 4,8 (s, 2H, 1-H); 5,5 (s, 1H, 3-H); 5,6 (s, 1H, 3-H); 8,0 (t, 1H, Aromaten-H); 8,4 (d, 1H, Aromaten-H); 8,7 (m, 2H, Aromaten-H) ppm.

### Beispiel 10

26,2 g der Verbindung aus Beispiel 9 wurden in 300 g Methanol und 2 g Propionsäure gelöst. Man gab 5 g Raney-Nickel zu und hydrierte bei 40°C und einem Wasserstoffdruck von 2 bar. Nach beendeter Wasserstoff-Aufnahme wurde vom Katalysator abfiltriert und die Mutterlauge vom Lösungsmittel befreit. Es verblieben 22,5 g der Verbindung der Formel 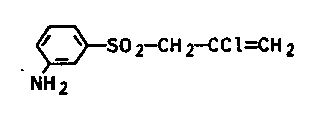
die ohne weitere Reinigung zu Farbstoffsynthesen verwendet werden kann.

Analog den Beispielen 9 und 10 werden die in der folgenden Tabelle 2 aufgeführten Verbindungen der Formel 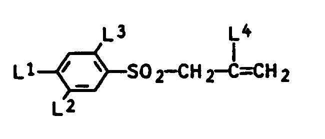
erhalten.

**Tabelle 2**

| Bsp. Nr. | L¹ | L² | L³ | L⁴ |
|---|---|---|---|---|
| 11 | H | NH₂ | H | Br |
| 12 | H | NH₂ | Cl | Cl |
| 13 | H | NH₂ | Cl | Br |
| 14 | Cl | NH₂ | H | Cl |
| 15 | Cl | NH₂ | H | Br |
| 16 | H | NH₂ | CH₃ | Cl |
| 17 | H | NH₂ | CH₃ | Br |
| 18 | H | NH₂ | N(CH₃)₂ | Cl |
| 19 | H | NH₂ | N(CH₃)₂ | Br |
| 20 | H | NH₂ | NHCH₂CH₂OH | Cl |
| 21 | H | NH₂ | NHCH₂CH₂OH | Cl |
| 22 | H | NH₂ | OCH₃ | Cl |

### Beispiel 23

160 g (0,64 mol) 2-Acetylaminonaphthalin-6-sulfinsäure wurden bei einem pH-Wert von 6 in 1000 ml Wasser gelöst und zu einer Lösung von 78,5 g (0,71 mol) 2,3-Dichlorprop-1-en und 80 ml THF gegeben. Das Reaktionsgemisch wurde etwa 10 Stunden auf ca. 70°C erwärmt. Nach beendeter Umsetzung (DC-Kontrolle) wurde der entstandene Niederschlag bei ca. 10°C abgesaugt, mit Wasser gewaschen und getrocknet. Es verblieben 163,3 g einer Verbindung der Formel 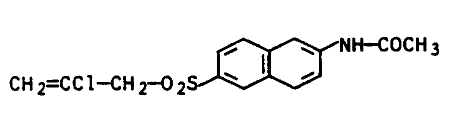
mit einem Schmelzpunkt von 148 bis 152°C.
- ¹H-NMR: (D₆-DMSO): δ =: 2,16 (s, 3H, CH₃); 3,35 (t, 1H, C≡XH); 4,60 (d, 2H, CH₂); 7,80 - 8,55 (m, 6H, aromat. H); 10,33 (s, 1H, NH) ppm.

### Beispiel 24

160 g (0,5 mol) der in Bsp. 23 beschriebenen Verbindung wurden mit 740 g 10 gew.-%iger Salzsäure zum Sieden erhitzt. Nach beendeter Entacetylierung (DC-Kontrolle) wurde das Reaktionsgemisch auf ca. 10°C abgekühlt und der Niederschlag abgesaugt. Es entstanden 146 g einer Verbindung der Formel 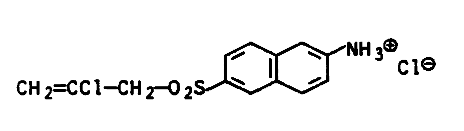
- ¹H-NMR (D₆-DMS0): δ =: 4,63 (s, 2H, CH₂); 5,47 (s, 1H, C=CH); 5,53 (s, 1H, C=CH); 7,55 - 8,53 (m, 6H, aromat. H); 9,43 (s, 3H, NH₃^{⊕}) ppm.

### Beispiel 25

160 g (0,64 mol) 2-Acetylaminonaphthalin-6-sulfinsäure wurden in 1000 ml Wasser bei einem pH-wert von 6 gelöst und zu einer Lösung aus 84,1 g (0,7 mol) 3-Bromprop-1-in und 85 ml THF getropft. Nach der Zugabe wurde das Reaktionsgemisch bis zur vollständigen Umsetzung (DC-Kontrolle) auf 40 bis 60°C erwärmt. Der entstandene Niederschlag wurde bei ca. 10°C abgesaugt, mit Wasser gewaschen und getrocknet. Es entstanden 166,4 g 2-Acetylamino-6-(prop-1-in-3-ylsulfonyl)naphthalin der Formel 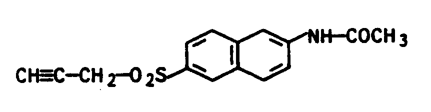
mit einem Schmelzpunkt von 230 bis 238°C.
- ¹H-NMR (D₆-DMSO): δ =: 2,16 (s, 3H, CH₃); 3,35 (t, 1H, C≡CH); 4,6 (d, 2H, CH₂); 7,8 - 8,55 (m, 6H, aromat. H); 10,33 (s, 1H, NH) ppm.

### Beispiel 26

59 g (0,26 mol) 2-Acetylamino-6-(prop-1-in-3-ylsulfonyl)naphthalin wurden in 400 ml 10 gew.-%iger Salzsäure 2 Stunden auf 100°C erhitzt. Die heiße Lösung wurde klärfiltriert, der in der Kälte sich bildende Niederschlag bei ca. 10°C abgesaugt, gewaschen und getrocknet. Es resultierten 54 g 2-Amino-6(prop-1-in-3-ylsulfonyl)naphthalin-hydrochlorid (Schmp. 208 bis 213°C). 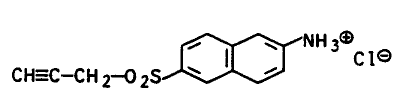
- ¹H-NMR (D₆-DMSO): δ =: 3,5 (1H, C≡CH); 4,73 (2H, CH₂); 7,7 - 8,7 (6H, aromat. H); 8,85 (3H, NH₃^{⊕}) ppm.
- ¹³C-NMR (D₆-DHSO): δ =: 7,1 (CH₂); 72,8 (C≡C); 78,1 (C≡CH); 118,6; 122,8; 124,0; 128,6; 129,3; 129,9; 131,3; 134,4; 135,2; 135,8; (aromat. C) ppm.

### Beispiel 27

199 g (1,0 mol) 4-Acetylaminobenzolsulfinsäure wurden in 1000 ml Wasser bei einem pH-Wert von 6 gelöst, zu einer Lösung aus 140 g (1,1 mol) 3-Bromprop-1-in und 200 ml THF bei 40 bis 50°C getropft und bis zur vollständigen Umsetzung bei dieser Temperatur gehalten (DC-Kontrolle). Der entstandene Niederschlag wurde bei ca. 10°C abgesaugt, mit Wasser neutral gewaschen und getrocknet. Es wurden 205 g 4-Acetylamino-1-(prop-1-in-3-ylsulfonyl)benzol erhalten. (Schmp. 175 bis 179°C). 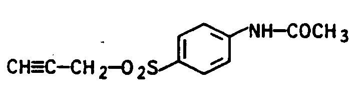
- ¹H-NMR (D₆-DMSO): δ =: 2,13 (3H, CH₃); 3,4 (1H, C≡CH); 4,47 (2H, CH₂); 7,83 - 7,92 (4H, aromat.); 10,45 (1H, NH) ppm.
- ¹³C-NMR (D₆-DMSO): δ =: 4,1 (CH₃); 47,2 (CH₂); 73,0 (C≡C); 77,8 (C≡CH); 118,4; 129,6; 131,3; 144,4 (aromat. C); 169,1 (C=0) ppm.

### Beispiel 28

205 g (0,865 mol) 4-Acetylamino-1-(prop-1-in-3-ylsulfonyl)benzol wurden in 1000 ml 10 gew.-%iger Salzsäure ca. 1,5 Stunden zum Sieden erhitzt. Nach vollständiger Entacetylierung (DC-Kontrolle) wurde das Reaktionsgemisch klärfiltriert und auf ca. 10°C abgekühlt. Der entstandene Niederschlag wurde abgesaugt, mit etwas 10 gew.-%iger Salzsäure nachgewaschen und getrocknet. Es verblieben 168 g 4-Amino-1-(prop-1-in-3-ylsulfonyl)benzolhydrochlorid. (Schmp. 225 bis 229°C). 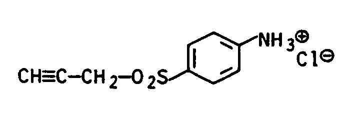
- ¹H-NMR (D₆-DMSO): δ =: 3,37 (t, 1H, C≡CH); 4,33 (d, 2H, CH₂); 6,90 - 7,65 (4H, aromat. H); 8,5 (3H, NH₃^{⊕}) ppm.
- ¹³C-NMR (D₆-DMS0): δ =: 47,6 (CH₂); 73,4 (C≡C); 77,3 (C≡CH); 114,4; 125,1; 130,1; 151,4 (aromat. C) ppm.

### Beispiel 29

99,5 g (0,5 mol) 3-Acetylaminobenzolsulfinsäure wurden in 500 ml Wasser bei einem pH-wert von 6,5 gelöst und zu einem Gemisch aus 65,5 g (0,55 mol) 3-Bromprop-1-in und 70 ml THF getropft. Zur vollständigen Umsetzung (DC-Kontrolle) wurde das Reaktionsgemisch auf 40 bis 50°C erwärmt. Der entstandene Niederschlag wurde bei 10°C abgesaugt, mit Wasser neutral gewaschen und getrocknet. Man isolierte 83 g der Verbindung der Formel 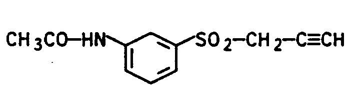
mit einem Schmelzpunkt von 159 bis 165°C.
- H-NMR (D₆-DMS0): δ =: 2,12 (s, 3H, CH₃); 3,43 (t, 1H, C≡CH); 4,53 (d, 2H, CH₂); 7,63 - 8,27 (m, 4H, aromat. H); 10,38 (s, 1H, NH) ppm.

### Beispiel 30

71,1 g (0,3 mol) der Verbindung aus Beispiel 29 wurden in 300 ml 10 gew.-%iger Sälzsäure zum Sieden erhitzt. Nach beendeter Entacetylierung wurde das Reaktionsgemisch auf 10°C abgekühlt und der entstandene Niederschlag abgesaugt. Man erhielt 54,2 g einer Verbindung, die der Formel 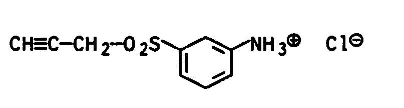
entspricht und einen Schmelzpunkt von 213 bis 217°C besitzt.
- ¹H-NMR (D₆-DMSO): δ =: 3,45 (t, 1H, C≡CH); 4,60 (d, 2H, CH₂); 7,55 - 7,72 (m, 4H, aromat. H); 9,38 (s, 3H, NH₃^{⊕}) ppm.

### Beispiel 31

119,5 g (0,5 mol) 3-Acetylamino-4-methoxybenzolsulfinsäure wurden analog Beispiel 25 mit 65,5 g (0,55 mol) 3-Bromprop-1-in umgesetzt. Es resultierten 97,7 g einer Verbindung der Formel 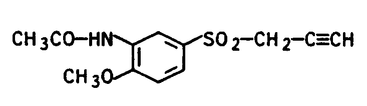
mit einem Schmelzpunkt von 140 bis 144°C.
- ¹H-NMR (D₆-DMSO): δ =: 2,15 (s, 3H, CH₃); 3,28 (t, 1H, CH); 3,95 (s, 3H, 0CH₃); 4,35 (d, 2H, CH₂); 7,27 - 8,63 (m, 3H, aromat. H); 9,28 (s, 1H, NH) ppm.

### Beispiel 32

79,2 g (0,3 mol) der Verbindung aus Beispiel 31 wurden analog Beispiel 26 entacetyliert. Es verblieben 63 g eines Feststoffs, der der Formel 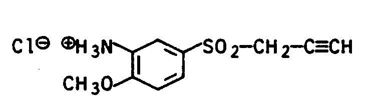
entspricht und einen Schmelzpunkt von 209 bis 214°C besitzt.
- ¹H-NMR (D₆-DMS0): δ =: 3,45 (t, 1H, C≡CH); 3,95 (s, 3H, CH₃) ; 4,47 (d, 2H, CH₂); 7,30 - 7,77 (m, 4H, aromat. H); 9,15 (s, 3H, NH₃^{⊕}) ppm.

### Beispiel 33

563 g (1,42 mol) 4-(2-Acetylaminoethyl)benzolsulfinsäure wurden analog Beispiel 25 mit 190,5 g (1,6 mol) 3-Bromprop-1-in umgesetzt. Man isolierte 307,5 g der Verbindung der Formel 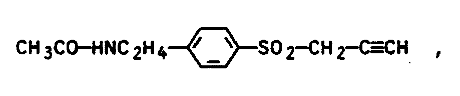
deren Schmelzpunkt 117 bis 118°C beträgt.
- ¹H-NMR (D₆-DMS0): δ =: 1,78 (s, 3H, CH₃); 2,87 (t, 2H, CH₂); 3,30 (t, 1H, C≡CH); 3,35 (t, 2H, CH₂); 4,45 (d, 2H, C≡C-CH₂); 7,48, 7,52, 7,87, 7,90 (4H, aromat. H); 7,82 (s, 1H, NH) ppm.

### Beispiel 34

302 g (1,14 mol) der Verbindung aus Beispiel 33 wurden mit 1000 ml 10 gew.-%iger Salzsäure zum Sieden erhitzt. Nach beendeter Umsetzung (DC-Kontrolle) wurde das Reaktionsgemisch bis zur Trockene eingedampft. Es verblieben 266 g einer Verbindung, die der unten stehenden Formel entspricht. 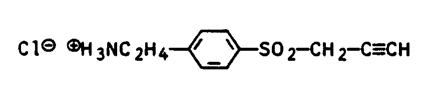
- ¹H-NMR (D₆-DMSO): δ =: 3,13 (2H, CH₂); 3,37 (3H, CH₂ und C≡CH); 4,47 (2H, C≡C-CH₂); 7,60, 7,63, 7,92, 7,95 (4H, aromat. H); 9,32 (3H, NH₃) ppm.

Anstelle des in den Beispielen 25, 27, 29, 31 und 33 verwendeten 3-Bromprop-1-in kann ebenso 3-Chlorprop-1-in verwendet werden.

In analoger Weise können die in der folgenden Tabelle 3 aufgeführten Verbindungen der Formel 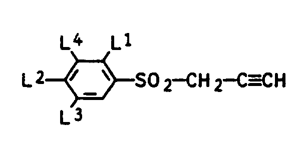
erhalten werden.

**Tabelle 3**

| Bsp. Nr. | L¹ | L² | L³ | L⁴ |
|---|---|---|---|---|
| 35 | H | CH₂-NHCOCH₃ | H | H |
| 36 | H | CH₂-NH₂ | H | H |
| 37 | NHCOCH₃ | H | H | H |
| 38 | NH₂ | H | H | H |
| 39 | H | CH₃ | NHCOCH₃ | H |
| 40 | H | CH₃ | NH₂ | H |
| 41 | H | NHCOCH₃ | Cl | H |
| 42 | H | NH₂ | Cl | H |
| 43 | H | NHCOCH₃ | Cl | CH₃ |
| 44 | H | NH₂ | Cl | CH₃ |
| 45 | H | CH₃ | NHCOCH₃ | Cl |
| 46 | H | CH₃ | NH₂ | Cl |
| 47 | Cl | NHCOCH₃ | Cl | H |
| 48 | Cl | NH₂ | Cl | H |
| 49 | NHCOCH₃ | H | Cl | Cl |
| 50 | NH₂ | H | Cl | Cl |
| 51 | NHCOCH₃ | H | CH₃ | CH₃ |
| 52 | NH₂ | H | CH₃ | CH₃ |

Analog den Beispielen 1 und 2 können weitere Verbindungen der Formel 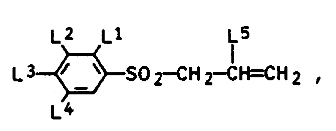
die in der folgenden Tabelle 4 aufgeführt sind, erhalten werden.

**Tabelle 4**

| Bsp. Nr. | L¹ | L² | L³ | L⁴ | L⁵ |
|---|---|---|---|---|---|
| 53 | H | CH₂-NHCOCH₃ | H | H | Cl |
| 54 | H | CH₂-NH₂ | H | H | Cl |
| 55 | NHCOCH₃ | H | H | H | Cl |
| 56 | NH₂ | H | H | H | Cl |
| 57 | H | CH₃ | NHCOCH₃ | H | Cl |
| 58 | H | CH₃ | NH₂ | H | Cl |
| 59 | H | NHCOCH₃ | Cl | H | Cl |
| 60 | H | NH₂ | Cl | H | Cl |
| 61 | H | NHCOCH₃ | Cl | CH₃ | Cl |
| 62 | H | NH₂ | Cl | CH₃ | Cl |
| 63 | H | CH₃ | NHCOCH₃ | Cl | Cl |
| 64 | H | CH₃ | NH₂ | Cl | Cl |
| 65 | Cl | NHCOCH₃ | Cl | H | Cl |
| 66 | Cl | NH₂ | Cl | H | Cl |
| 67 | NHCOCH₃ | H | Cl | Cl | Cl |
| 68 | NH₂ | H | Cl | Cl | Cl |
| 69 | NHCOCH₃ | H | CH₃ | CH₃ | Cl |
| 70 | NH₂ | H | CH₃ | CH₃ | Cl |

Anstelle des in den Beispielen 1 und 2 und 53 bis 70 verwendeten 2,3-Dichlorprop-1-en kann ebenso 2,3-Dibromprop-1-en verwendet werden, wobei entsprechende Verbindungen mit L⁴ = Br erhalten werden.

### Beispiel 71

24 g (0,1 mol) 4-Acetylamino-1-(prop-1-in-3-ylsulfonyl)benzol (Beispiel 32) wurden in 100 ml THF gelöst, mit 1 g (0,01 mol) Triethylamin versetzt und zum Sieden erhitzt. Nach beendeter Isomerisierung (DC-Kontrolle) wurde das Lösungsmittel abgezogen und der Rückstand aus Essigester umkristallisiert. Es wurden 20 g einer Verbindung erhalten, die der Formel 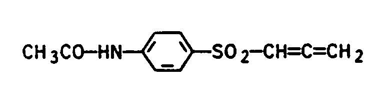
entspricht.
- ¹H-NMR (D₆-DMS0): δ =: 2,12 (s, 3H, CH₃); 5,68 (d, 2H, CH₂); 6,70 (t, 1H, CH); 7,80-7,88 (m, 4H, aromat. H); 10,33 (s, 1H, NH) ppm.

### Beispiel 72

26,7 g (0,1 mol) 3-Acetylamino-4-methoxy-1-(prop-1-in-3-ylsulfonyl)benzol (Beispiel 31) wurden analog Beispiel 71 mit 0,01 mol Triethylamin umgesetzt. Es resultierten 24 g einer Verbindung, die der Formel 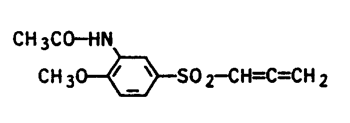
entspricht.
- ¹H-NMR (D₆-DMS0): δ =: 2,15 (s, 3H, CH₃); 3,97 (s, 3H, OCH₃); 5,68 (d, 2H, CH₂); 6,85 (t, 1H, CH); 7,25, 7,57, 8,58 (3H, aromat., H), 9,32 (s, 1H, NH) ppm.

### Beispiel 73

27,4 g (0,1 mol) 4-Acetylamino-1-(2-chlorprop-1-en-3-ylsulfonyl)benzol (Beispiel 1) wurden In 150 ml THF gelöst, mit 10,1 g (0,1 mol) Triethylamin versetzt und zum Sieden erhitzt. Nach beendeter Umsetzung (DC-Kontrolle) wurde der Niederschlag abfiltriert, das Filtrat eingeengt und der Rückstand aus Essigester umkristallisiert. Es entstanden 19 g 4-Acetylamino-1-(propa-1,2-dien-3-ylsulfonyl)benzol, das mit der in Beispiel 71 beschriebenen Verbindung identisch ist.

Analog Beispiel 72 und 73 können die in der folgenden Tabelle 5 aufgeführ ten Verbindungen der Formel 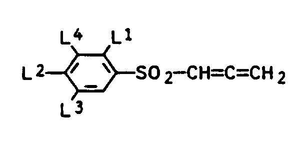
erhalten werden.

**Tabelle 5**

| Bsp. Nr. | L¹ | L² | L³ | L⁴ |
|---|---|---|---|---|
| 74 | H | CH₂-NHCOCH₃ | H | H |
| 75 | NHCOCH₃ | H | H | H |
| 76 | H | CH₃ | NHCOCH₃ | H |
| 77 | H | NHCOCH₃ | Cl | H |
| 78 | H | NHCOCH₃ | Cl | CH₃ |
| 79 | H | CH₃ | NHCOCH₃ | Cl |
| 80 | CL | NHCOCH₃ | Cl | H |
| 81 | NHCOCH₃ | H | Cl | Cl |
| 82 | NHCOCH₃ | H | CH₃ | CH₃ |
| 83 | H | CH₃CONH-CH₂CH₂ | H | H |

Die aufgeführten Acetylaminoverbindungen können analog den Beispielen 2, 24 und 26 in die entsprechenden Aminoverbindungen übergeführt werden.

## Patentansprüche

1. Sulfonylverbindungen der Formel I 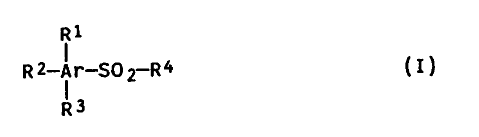 in freier Form oder in Form ihrer Salze, worin
Ar den Rest eines Benzol- oder Naphthalinringes,
R¹ und R² gleich oder verschieden sind und unabhängig voneinander jeweils Wasserstoff, gegebenenfalls durch Amino oder C₁-C₄-Alkanoylamino substituiertes C₁-C₄-Alkyl, gegebenenfalls substituiertes Phenyl, C₁-C₄-Alkoxy, Carboxyl, C₁-C₄-Alkoxycarbonyl, Cyano, Halogen oder Hydroxysulfonyl,
R³ Amino, C₁-C₄-Alkanoylamino oder Benzoylamino und
R⁴ einen Rest der Formel 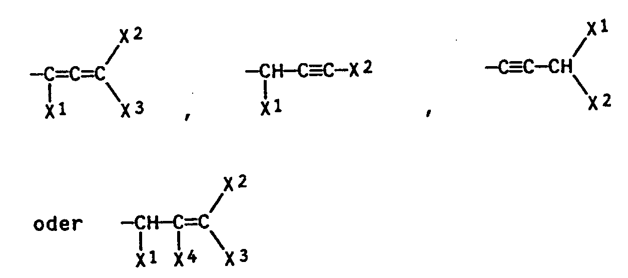 bedeuten, worin
X¹, X² und X³ gleich oder verschieden sind und unabhängig voneinander jeweils für Wasserstoff, gegebenenfalls durch Hydroxy oder C₁-C₄-Alkoxy substituiertes C₁-C₄-Alkyl oder gegebenenfalls substituiertes Phenyl und
X⁴ für eine unter alkalischen Reaktionsbedingungen abspaltbare Gruppe stehen.

2. Sulfonylverbindungen nach Anspruch 1, dadurch gekennzeichnet, daß R⁴ einen Rest der Formel 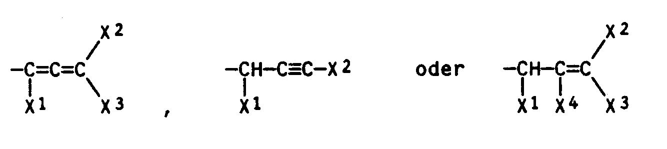 bedeutet, worin
X¹, X² und X³ jeweils für Wasserstoff und
X⁴ für Halogen stehen.

3. Verfahren zur Herstellung von Sulfonylverbindungen gemäß Anspruch 1, die der Formel Ia 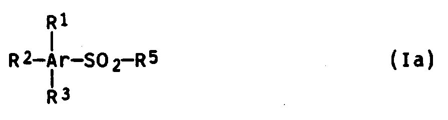 gehorchen, worin Ar, R¹, R² und R³ jeweils die in Anspruch 1 genannte Bedeutung besitzen und R⁵ einen Rest der Formel 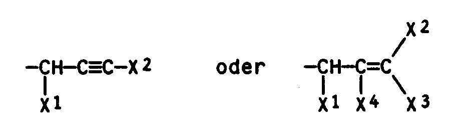 bedeutet, worin X¹, X², X³ und X⁴ jeweils die in Anspruch 1 genannte Bedeutung besitzen, durch Umsetzung von Arylsulfinsäuren der Formel II 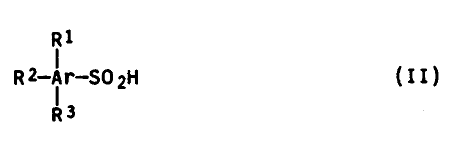 mit Halogenverbindungen der Formel IIIa oder IIIb 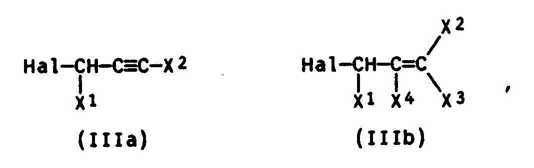 wobei Hal jeweils Halogen bedeutet und Ar, R¹, R², R³, X¹, X², X³ und X⁴ jeweils die in Anspruch 1 genannte Bedeutung besitzen, in wäßrigem Medium bei einer Temperatur von 20 bis 95°C, dadurch gekennzeichnet, daß man die Umsetzung bei einem pH-Wert von 2,5 bis 7 durchführt.

4. Verwendung von Sulfonylverbindungen gemäß Anspruch 1, die der Formel Ib 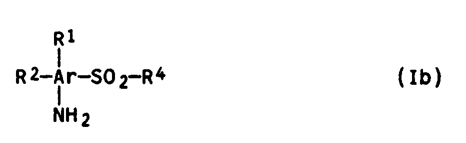 gehorchen, in der R¹, R² und R⁴ jeweils die in Anspruch 1 genannte Bedeutung besitzen, als Ankersystem und gegebenenfalls gleichzeitig als Diazokomponente bei der Herstellung von Reaktivfarbstoffen.

## Claims

1. A sulfonyl compound of the formula I 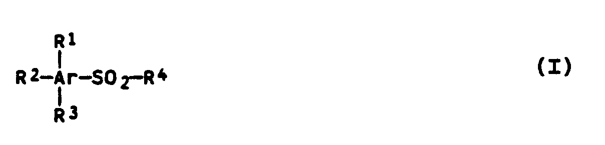 where
Ar is the radical of a benzene or naphthalene ring,
R¹ and R² are identical or different and each is independently of the other hydrogen, unsubstituted or amino- or C₁-C₄-alkanoylamino-substituted C₁-C₄-alkyl, substituted or unsubstituted phenyl, C₁-C₄-alkoxy, carboxyl, C₁-C₄-alkoxycarbonyl, cyano, halogen or hydroxysulfonyl,
R³ is amino, C₁-C₄-alkmoylamino or benzoylamino, and
R⁴ is a radical of the formula 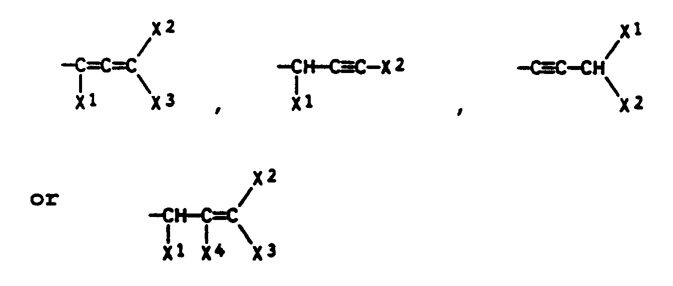 where
X¹, X² and X³ are identical or different and each is independently of the others hydrogen, unsubstituted or hydroxyl- or C₁-C₄-alkoxy-substituted C₁-C₄-alkyl or substituted or unsubstituted phenyl, and
X⁴ is a group which is detachable under alkaline reaction conditions,
in the free form or in the form of a salt thereof.

2. A sulfonyl compound as claimed in claim 1, wherein R⁴ is a radical of the formula 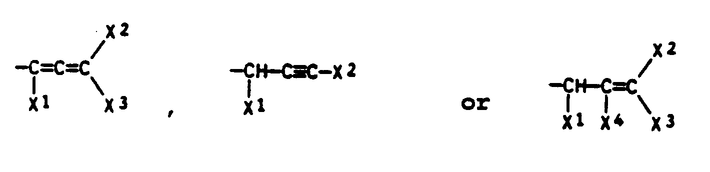 where
X¹, X² and X³ are each hydrogen, and
X⁴ is halogen.

3. A process for preparing a sulfonyl compound as claimed in claim 1 of the formula Ia 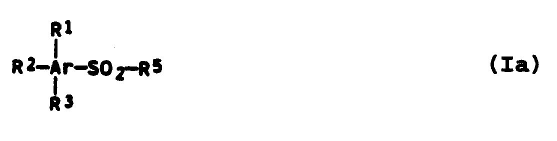 where Ar, R¹, R² and R³ are each as defined in claim 1 and R⁵ is a radical of the formula 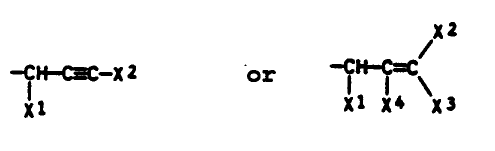 where X¹, X², X³ and X⁴ are each as defined in claim 1, by reacting an arylsulfinic acid of the formula II 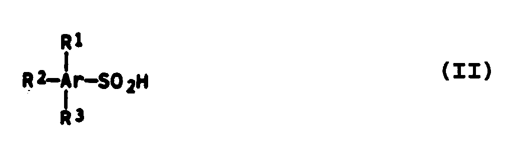 with a halogen compound of the formula IIIa or IIIb 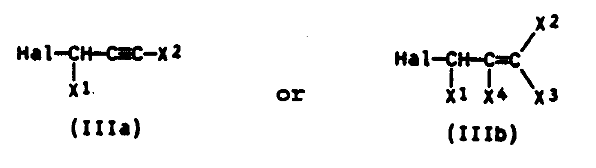 where Hal is in each case halogen and Ar, R¹, R², R³, X¹, X², X³ and X⁴ are each as defined in claim 1, in an aqueous medium at from 20 to 95°C, which comprises carrying out the reaction at a pH of from 2.5 to 7.

4. The use of a sulfonyl compound as claimed in claim 1 of the formula Ib 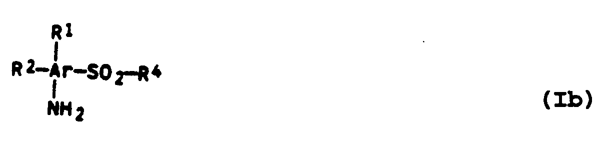 where R¹, R² and R⁴ are each as defined in claim 1, as reactive system and possibly also as diazo component in the synthesis of reactive dyes.

## Revendications

1. Dérivés sulfonylés de formule I 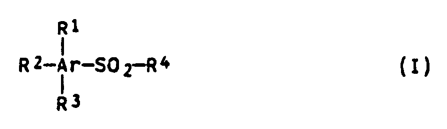 sous forme libre ou sous forme de leurs sels, dans laquelle
Ar représente le reste d'un noyau benzène ou naphtalène,
R¹ et R² sont identiques ou différents et représentent chacun indépendamment l'un de l'autre un atome d'hydrogène, un groupement alkyle en C₁-C₄ éventuellement substitué par un groupement amino ou alcanoylamino en C₁-C₄, un groupement phényle éventuellement substitué, un groupement alcoxy en C₁-C₄, un groupement carboxy, un groupement (alcoxy en C₁-C₄)carbonyle, un groupement cyano, un atome d'halogène ou un groupement hydroxysulfonyle,
R³ représente un groupement amino, alcanoylamino en C₁-C₄ ou benzoylamino et
R⁴ représente un reste de formule 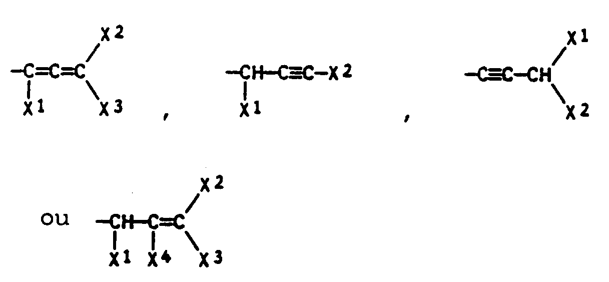 dans lesquelles
X¹, X² et X³ sont identiques ou différents et représentent chacun indépendamment les uns des autres un atome d'hydrogène, un groupement alkyle en C₁-C₄ éventuellement substitué par un groupement hydroxy ou alcoxy en C₁-C₄ ou un groupement phényle éventuellement substitué et
X⁴ représente un groupement qui peut être éliminé dans des conditions de réaction alcalines.

2. Composés sulfonylés selon la revendication 1, caractérisé en ce que R⁴ représente un reste de formule 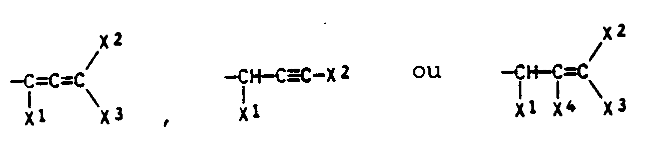 dans lesquelles
X¹, X² et X³ sont chacun mis pour un atome d'hydrogène et
X⁴ est mis pour un atome d'halogène.

3. Procédé de préparation de dérivés sulfonylés selon la revendication 1, qui ont la formule Ia 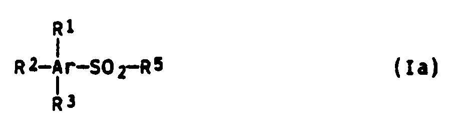 dans laquelle Ar, R¹, R² et R³ ont chacun la signification donnée dans la revendication 1 et R⁵ est un reste de formule 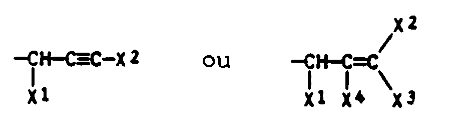 dans lesquelles X¹, X², X³ et X⁴ ont chacun la définition donnée dans la revendication 1,
par réaction d'acides arylsulfiniques de formule II 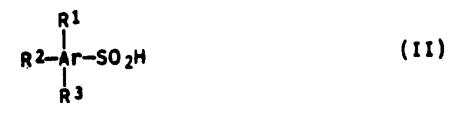 avec des composés halogénés de formule IIIa ou IIIb 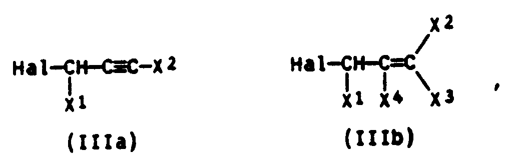 dans laquelle Hal représente à chaque fois un atome d'halogène et Ar, R¹, R², R^{3,} X¹, X², X³ et X⁴ ont chacun la définition donnée dans la revendication 1,
en milieu aqueux à une température de 20 à 95°C, caractérisé en ce qu'on effectue la réaction à un pH de 2,5 à 7.

4. Utilisation de composés sulfonylés selon la revendication 1, qui ont la formule Ib 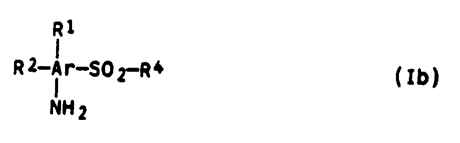 dans laquelle R¹, R² et R⁴ ont chacun la définition donnée dans la revendication 1, comme systèmes d'ancrage et éventuellement simultanément comme composants diazoïques lors de la préparation de colorants réactifs.
